# EUROPÄISCHE PATENTSCHRIFT

(11) **EP 0 982 010 B1**
(45) Veröffentlichungstag und Bekanntmachung des Hinweises auf die Patenterteilung: **22.12.2004**
(21) Anmeldenummer: 99250285.6
(22) Anmeldetag: 20.08.1999
(51) Int. Cl.: A61F 2/06

(54) **Ballonkatheter**
Balloon catheter
Cathéter à ballonnet

(30) Priorität: 21.08.1998 DE 19840701
(43) Veröffentlichungstag der Anmeldung: 01.03.2000
(73) Patentinhaber: BIOTRONIK Mess- und Therapiegeräte GmbH & Co Ingenieurbüro Berlin, 12359 Berlin (DE)
(72) Erfinder: Zedler, Stephan, Dipl.-Ing., 12309 Berlin (DE)
(74) Vertreter: Eisenführ, Speiser & Partner

(56) Entgegenhaltungen:
- EP-A- 0 770 366
- EP-A- 0 901 776
- WO-A-96/38109
- WO-A-98/07390
- US-A- 5 759 474

## Beschreibung

Die Erfindung betrifft einen Ballonkatheter zur Aufweitung von Gefäßverengungen und zur gleichzeitigen Einbringung eines bleibend verformbaren Stents in das aufzuweitende Gefäß, um dieses in aufgeweitetem Zustand zu stabilisieren, wobei der zur Aufnahme des Stents vorgesehene distale Bereich des Katheters einen Innenschlauch aufweist, der von dem nicht expandierten Stent umgeben ist, ein Ballon zwischen Stent und Innenschlauch angeordnet ist und der Innenschlauch an seinen Enden als Röntgenmarkierungen zwei auf diesen aufgebrachten Hülsen aufweist, welche aus aeinem röntgenpaquen Material bestehen und innerhalb des Ballons auf dem Innenschlauch vorgesehen sind.

Die Verengung oder der Verschluß von Gefäßen wird mittels der in den letzten Jahren zum Standardeingriff gewordenen perkutanen transluminalen koronaren Angioplastie (PTCA) behandelt. Dazu werden Katheter eingesetzt, die an ihrem distalen Ende einen zylindrischen Ballon aufweisen, der im inflatierten Zustand eine Konfigurationsänderung des Gefäßlumens bewirkt, in der Weise, daß die innere und die mittlere Schicht des Gefäßlumens in Längsrichtung einreißen und die verbleibende äußere Schicht so ausgestülpt wird, daß sie eine neue Gefäßwand bildet.

Die neue Gefäßwand wird dabei durch plastisch verformte Stents (Gefäßwandstützen) auf ihrem gewünschten Innendurchmesser gehalten. Der Stent befindet sich im nicht expandierten, gecrimpten Zustand auf dem Ballonabschnitt am distalen Ende des Katheters und wird an die Stelle der Gefäßverengung oder des - verschlusses geschoben. Nach der Balloninflation verbleibt der plastisch verformte Stent im Gefäß und der deflatierte Ballonkatheter wird wieder entfernt.

Es sind Ballonkatheter bekannt, die durch ihre Ausgestaltung sicherstellen sollen, daß die Gefäßwände beim Einfüh- ren des Katheters möglichst wenig beschädigt werden. Dazu ist ein Schutzkatheter über dem eigentlichen Katheter vorgesehen, auf dem der Stent zusammengedrückt gehalten ist. Durch diesen Schutzkatheter wird auch eine Verschiebung des Stents aus dem Ballonbereich des Katheters heraus verhindert, die sonst eine ordnungsgemäße Inflation und - so- mit auch die gewünschte plastische Verformung des Stents verhindern würde.

Der Nachteil dieses zusätzlichen Katheters liegt in der deutlichen Zunahme des Außendurchmessers der gesamten Einrichtung, der Abnahme der Flexibilität und der eventuell nötigen Vordilatation der Engstelle zur Einbringung der Gefäßstütze, weil ohne diese, mit einem zusätzlichen Ballonkatheter vorzunehmende Vordilatation, der größere Außendurchmesser der gesamten Einrichtung mit Schutzkatheter nicht problemlos vor Ort zu plazieren wäre. Gerade in engen Gefäßen oder an schwierig zugänglichen Stellen, beispielsweise wegen stark gekrümmter Gefäßverläufe, wirken sich diese Nachteile besonders aus. Aus der deutschen Offenlegungsschrift DE 195 40 084 A1 ist eine Vorrichtung bekannt, die ohne einen zusätzlichen Schutzkatheter auskommt, weil am distalen Ende des Katheters ein den gecrimpten Stent abschirmendes Stützteil vorgesehen ist, welches in Richtung der Kathetereinführung, gewissermaßen als Schild, den Stent in radialer Richtung überragt. Durch diese Anordnung wird verhindert, daß der Stent gegen Hindernisse stoßen kann, da der Außendurchmesser des auf dem Ballonbereich zusammengedrückten Stents geringer ist, als der der Stützteile.

Die Nachteile dieser Anordnung bestehen vor allem darin, daß die Stützteile auf dem Innenschlauch den Durchmesser der gesamten Einrichtung stark vergrößern. Zwar nicht auf der gesamten Länge des einzubringenden Stents wie bei der Lösung mit zusätzlichem Schutzkatheter, doch an Gefäßengstellen ist gleichwohl eine erhebliche Aufweitung notwendig. Der in dem Zwischenraum zwischen den Stützteilen eingebettete Stent liegt direkt an der Ballonhülle auf dem Innenschlauch des Katheters an. Der Stent muß sich dadurch genau so stark verformen wie der Katheter selbst - es besteht deshalb keine wesentliche Dämpfung zwischen Innenschlauch und Stent. Dies birgt zusätzlich die Gefahr, dass bei Einbringung in enge gekrümmte Gefäßverläufe der Stent schon vorher, d.h. bei der Einbringung plastisch verformt wird und daher bei der Inflation nicht mehr das gewünschte Ausdehnungsverhalten zeigt.

Aus der europäischen Patentanmeldung EP 0 820 784 A2 ist weiterhin ein Ballonkatheter bekannt, der den Stent dadurch schützt, dass ein Innen- und ein Außenrohr vorgesehen sind. Das den Stent tragende Außenrohr weist einen, am proximalen Ende befestigten, Schlauch auf, der den darunterliegenden Stent schützt. Am distalen Ende wird der Schlauch außen radial entlang der Längsachse des Außenrohres wieder zurückgeführt, den Stent und den "Hinweg" des Schlauchs in Gegenrichtung von distal nach proximal überstülpend. Das Innenrohr weist proximal die Befestigung des anderen Endes des Schlauches auf, und zwar in der Weise, dass bei Herausziehen des Innen- aus dem Außenrohr die in Längsrichtung existierende Auffaltung des Schlauches entstülpt und der Stent freigelegt wird. Nach der Positionierung und dem Freilegen des Stents durch Herausziehen des Innenrohres, kann der Ballon inflatiert werden. Auch bei diesem Katheter ist eine besondere Zunahme des Außendurchmessers der Gesamteinrichtung zu verzeichnen. Der Nachteil des großen Durchmessers durch zwei ineinander liegende Rohre und zwei Wandstärken des überstülpenden Schlauches ist hier ganz besonders gegeben, so dass die Flexibilität des Katheters deutlich eingeschränkt ist.

Aus der WO 98/07390 ist ein Ballonkatheter bekannt, der zwischen einem Innenschlauch und einem expandierbaren Ballon einen weiteren, gewellten Schlauch besitzt, der sich über auf den Innenschlauch angebrachte Hülsen hinaus erstreckt und in Längsrichtung zwischen den Hülsen ein Luftpolster einschließt, welches bei nicht expandiertem Stent komprimiert ist, wobei der Schlauch in diesem Zustand im Bereich der Hülsen einen größeren Außendurchmesser hat, als in seinem zwischen den Hülsen liegenden Längsabschnitt.

Der Erfindung liegt deshalb die Aufgabe zugrunde, einen Ballonkatheter anzugeben, der bei guter Sicherheit gegen Verschieben des Stents, beim Einführen dennoch eine hohe Flexibilität und ein möglichst geringer Durchmesser des Katheters realisiert werden können. Dabei soll der nicht expandierte Stent optimal am deflatierten Ballon anliegen.

Diese Aufgabe wird erfindungsgemäß durch einen Ballonkatheter der eingangs genannten Art gelöst, der sich dadurch auszeichnet, dass zwischen Innenschlauch und äußerem Ballon ein ein zusätzliches Plateau bildender Schlauch aus einem weichelastischen Material als Zwischenlage derart vorgesehen ist, dass er sich in Längsrichtung bis über die die Röntgenmarkierungen bildenden Hülsen erstreckt.

Die Erfindung schließt die technische Lehre ein, dass der Stent, eingedrückt in eine weiche Materialunterlage, deutlich besser am deflatierten Ballon anliegt. Das zwischen dem die Zwischenlage bildenden Schlauch und dem Innenschlauch des Katheters eingeschlossene Luftpolster - resultierend aus dem radialen Abstand der dazwischenliegenden Röntgenmarkierungsringe - bildet eine Bettung für den gecrimpten Stent. Das weiche Material des die Zwischenlage bildenden Schlauches bewirkt dabei einerseits ein gutes Anliegen des Stents und läßt andererseits durch seine Nachgiebigkeit auch eine optimale Faltenbildung des deflatierten Ballons zu, so daß dieser auch vor Beschädigung durch lokale Überbeanspruchung geschützt ist.

Besonders vorteilhaft dabei ist auch, daß der gecrimpte Stent beim Voranschieben des Katheters sicher gehalten und damit gegen ein Verschieben in Längsrichtung gesichert ist.

Damit kann er insbesondere beim Einbringen nicht verloren gehen.

Ebenso werden die bei der Verfolgung enger Gefäßkrümmungen nötigen Verbiegungen des Katheters nicht direkt und in voller Stärke an den Stent weitergegeben und die Ungleichmäßigkeiten der Faltung des Ballons werden teilweise ebenfalls vom dem die Zwischenlage bildenden Schlauch aufgenommen. Dadurch wird verhindert, daß Bereiche stärkerer Verbiegung des Stents schon auf dem Weg zur Implantierungsstelle entstehen, die beim Aufblasen des Stents zu einer ungleichmäßigen Stentdehnung führen können.

Der die Zwischenlage bildende Schlauch ist vorzugsweise nur an seinem distalen Ende an dem Katheter, insbesondere an dem Innenschlauch befestigt. Dadurch wird verhindert, daß durch Schlauchabschnitt sich beim vor- und zurückbewegen des Katheters aufwölbt oder wellt.

In einer anderen vorteilhaften Variante der Erfindung ist vorgesehen, die auf dem Innenschlauch des distalen Endes des Katheters plazierten Röntgenmarkierungsringe mit dem in Längsrichtung des Katheters verlaufenden, die Zwischen- lage bildende Schlauch einzuhüllen, der an seinem distalen Ende mit dem Innenschlauch des Katheters verschweißt ist. Der die Zwischenlage bildende Schlauch besteht aus einem gummiartigen, weichelastischen Material, vorzugsweise mit einer Shorehärte von 35 D, und ist zum proximalen Ende hin in Längsrichtung offen. Der Ballon des Katheters umhüllt Innenschlauch, Röntgenmarkierungsringe und den die Zwischenlage bildende Schlauch.

Der Stent läßt sich durch Zusammendrücken auf dem Ballon- bereich gut plazieren, paßt in seiner Länge in den Abstand zwischen den beiden Röntgenmarkierurigsringen und verdrängt dabei das Luftpolster zwischen der die Zwischenlage bildenden Schlauch und dem Innenschlauch.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
- Figur 1 als bevorzugtes Ausführungsbeispiel der Erfindung einen Ballonkatheter in Längsschnittsdarstellung und
- Figur 2 den Ballonkatheter gemäß Figur 1 mit dem gecrimpten Stent in vollständig zum Einbringen vorgefertigter Komplettausführung.

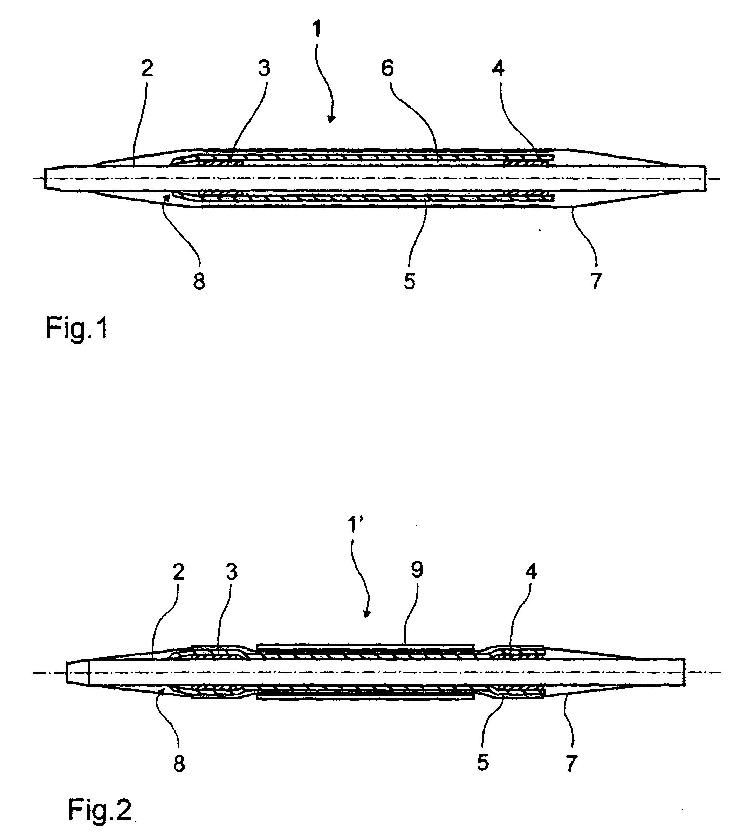

In Figur 1 - bei der die distale Richtung nach links weist - ist ein Ballonkatheter 1 gezeigt, welcher auf einem langgetreckten Innenschlauch 2 angebrachten Hülsen 3, 4 aus einem röntgenopaquen Material als sogenannte Röntgenmarkierungen aufweist. Darüber ist ein den Abstand zwischen den beiden Hülsen 3, 4 überbrückender, eine Zwischenlage bildender Schlauch 5 vorgesehen, welcher am distalen Ende mit dem Innenschlauch 2 an der Stelle 7 verschweißt ist. Der Schlauch 5 besteht aus einem gummiartigen weichelastischen Material von der Shorehärte 35 D. Dieses weist eine haftungsfähige Oberfläche auf, die sich leicht komprimieren läßt.

Dadurch, daß der Innendurchmesser des die Zwischenlage bildenden Schlauchs in etwa dem Außendurchmesser der die Röntgenmarkierungen bildenden Hülsen entspricht, wird durch den radialen Abstand zwischen Innenschlauch 2 und dem die Zwischenlage bildenden Schlauch 5 ein Luftpolster 6 eingeschlossen, welches die Nachgiebigkeit bzw. Kompressibilität der Anordnung vergrößert. Ein zusammengefalteter Ballon 7 umhüllt die vorgenannten Elemente und ist durch Zufuhr eines flüssigen Mediums (physiologische Kochsalzlösung). Auf die in Figur 1 dargestellte Anordnung wird der in das Gefäß einzubringende Stent aufgeschoben und gecrimpt, damit er einen möglichst geringen Durchmesser einnimmt.

In Figur 2 ist der erfindungsgemäße Ballonkatheter 1 in fertig montierter Ausführung 1 dargestellt. Dabei ist der Stent 9 in einer fertig montierten Position wiedergegeben.

Das Luftpolster zwischen dem die Zwischenlage bildenden Stent und dem Innenschlauch ist komprimiert. Dieser ist so auf den Ballon 7 und in den die Zwischenlage bildenden Schlauch 5 zwischen den Röntgenmarkierungshülsen 3, 4 eingedrückt, daß das Luftpolster 6 aus dem Raum zwischen dem die Zwischenlage bildenden Schlauch und Innenschlauch verdrängt ist. Damit ist der Außendurchmesser des die Zwischenlage bildenden Schlauchs an den Außendurchmesser der Hülsen 3, 4 angepaßt, so daß sich eine bündige Oberfläche ergibt. Der gefaltete Ballon findet eine "weiche" Umgebung, welche die entstehenden Falten aufnimmt.

Der komprimierte Stent wird ebenfalls in seiner Strukturierung "weich" gehalten und ist durch die elastisch in seine Zwischenräume eindringenden Teile der Ballonoberfläche sicher vor einem unababsichtigten Verschieben auf seiner Unterlage geschützt. Zuzsätzlich ist er noch durch die Verdickungen gesichert, welche durch den auf den Hülsen 3, 4 auf liegenden Schlauch 5 nach dem Crimpen entstanden sind.

Die Anordnung gemäß Figur 2 bildet eine fertige, vormontierte Einheit 1', welche in dieser Form in die Klinik gelangt und nach Plazierung in dem zu therapierenden Gefäß durch ein flüssiges Medium, bevorzugt durch eine physiologische Kochsalzlösung, expandiert wird.

Die Erfindung beschränkt sich in ihrer Ausführung nicht auf die vorstehend angegebenen bevorzugten Ausführungsbeispiele. Vielmehr ist eine Anzahl von Varianten möglich, welche von der dargestellten Lösung auch bei grundsätzlich anders gearteten Ausführungen Gebrauch macht.

## Patentansprüche

1. Ballonkatheter (1) zur Aufweitung von Gefäßverengungen und zur gleichzeitigen Einbringung eines bleibend verformbaren Stents (9) in das aufzuweitende Gefäß, um dieses in aufgeweitetem Zustand zu stabilisieren, wobei der zur Aufnahme des Stents vorgesehene distale Bereich des Katheters einen Innenschlauch (2) aufweist, der von dem nicht expandierten Stent umgeben ist, ein Ballon (7) zwischen Stent (9) und Innenschlauch (2) angeordnet ist und der Innenschlauch an seinen Enden als Röntgenmarkierungen zwei auf diesen aufgebrachte Hülsen (3,4) aufweist, welche aus einem röntgenopaquen Material und innerhalb des Ballons (7) auf dem Innenschlauch (2) vorgesehen sind, wobei zwischen Innenschlauch (2) und äußerem Ballon (7) ein ein zusätzliches Plateau bildender Schlauch (5) aus einem weichelastischen Material als Zwischenlage derart vorgesehen ist,
dass er sich in Längsrichtung bis über die die Röntgenmarkierungen bildenden Hülsen (3, 4) erstreckt und in Längsrichtung zwischen den Hülsen (3, 4) ein Luftpolster einschließt, **dadurch gekennzeichnet, dass** das Luftpolster bei nicht expandiertem Stent komprimiert ist und der Schlauch (5) in diesem Zustand im Bereich der Hülsen (3, 4) einen größeren Außendurchmesser hat, als in seinem zwischen den Hülsen (3, 4) liegenden Längsabschnitt.

2. Ballonkatheter nach Anspruch 1, **dadurch gekennzeichnet, daß** der Innenddurchmesser des die Zwischenlage bildenden Schlauchs (5) dem Außendurchmesser der die Röntgenmarkierungen bildenden Hülsen (3, 4) angepaßt ist, so daß er in unbelastetem Zustand zum Innenschlauch (1) hin ein Luftpolster (6) einschließt.

3. Ballonkatheter nach Anspruch 1 oder 2, **dadurch gekennzeichnet, daß** der die Zwischenlage bildende Schlauch (5) im wesentlichen eine Stärke aufweist, welche derjenigen der die Röntgenmarkierungen bildenden Hülsen (3, 4) entspricht.

4. Ballonkatheter nach einem der vorhergehenden Ansprüche, **dadurch gekennzeichnet, daß** der die Zwischenlage bildende Schlauch (5) mindestens an einem Ende über die die Röntgenmarkierung bildende Hülse (3) hinausreicht.

5. Ballonkatheter nach einem der vorhergehenden Ansprüche, **dadurch gekennzeichnet, daß** der die Zwischenlage bildende Schlauch (5) nur an seinem distalen Ende an dem Katheter insbesondere an dem Innenschlauch (2) befestigt ist.

6. Ballonkatheter nach einem der vorhergehenden Ansprüche, daß die die Röntgenmarkierungen bildenden Hülsen (3, 4) einen luftdichten Abschluß bilden, so daß ein zwischen Innenschlauch (2) und dem äußeren, die Zwischenlage bildenden Schlauch (5) verbleibendes Luftvolumen ein elastisches Luftkissen (6) bildet, welches beim Crimpen des Stents (9) im Rahmen der Herstellung einen Widerhalt für ein kontrolliertes Ausweichen des Ballons (7) bildet.

7. Ballonkatheter nach einem der vorhergehenden Ansprüche, **dadurch gekennzeichnet, daß** das Luftkissen (6) sich in Längsrichtung zwischen den Röntgenmarkierungsringen (3,4) befindet und radial außerhalb des Innenschlauches (2) und der Röntgenmarkierungsringe (3, 4), aber innerhalb des Ballons (7) und des außen liegenden Stents (9) vorgesehen ist.

8. Ballonkatheter nach einem der vorhergehenden Ansprüche, **dadurch gekennzeichnet, daß** der Innenschlauch (2), der Ballon (7), der die Zwischenlage bildendene Schlauch (5) und der auf diese Unterlage aufgecrimpte Stent (9) eine vormontierte Einheit (1) bilden.

9. Ballonkatheter nach einem der vorangehenden Ansprüche, **dadurch gekennzeichnet, daß** der die Zwischenlage bildende Schlauch (5) aus einem weichen und elastischen Material besteht, in das der Stent (9) beim Crimpen hineingedrückt ist.

10. Ballonkatheter nach einem der vorhergehenden Ansprüche, **dadurch gekennzeichnet, daß** der die Zwischenlage bildende Schlauch (5) eine Shorehärte von im wesentlichen 35 D aufweist.

11. Ballonkatheter nach einem der vorangehenden Ansprüche, **dadurch gekennzeichnet, daß** der die Zwischenlage bildende Schlauch (5) am distalen Ende mit dem Innenschlauch (2) des Katheters verschweißt ist.

12. Ballonkatheter nach einem der vorangehenden Ansprüche, **dadurch gekennzeichnet, daß** der Außendurchmesser des Katheters mit aufgecrimptem Stent (9) zwischen den Röntgenmarkierungsringen (3, 4) einen Wert aufweist, welcher im wesentlichen gleich groß oder kleiner ist als der Wert des Katheteraußendurchmessers an den Stellen der aufgebrachten Markierungsringe (3, 4).

13. Ballonkatheter nach einem der vorangehenden Ansprüche, **dadurch gekennzeichnet, daß** der die Zwischenlage bildende Schlauch (5) außerhalb des Ballons (7), der Röntgenmarkierungsringe (3, 4) und des Katheterinnenschlauchs (2) angebracht ist und der Stent (9) darauf zusammengedrückt ist.

## Claims

1. Balloon catheter (1) for expanding narrowings in vessels and for simultaneously inserting a permanently deformable stcnt (9) into the vessel to be expanded in order to stabilise this vessel in the expanded state, wherein the distal region of the catheter provided to receive the stent has inner tubing (2) which is surrounded by the non-expanded stent, a balloon (7) is disposed between the stent (9) and the inner tubing (2) and the inner tubing has, at its ends, two sleeves (3, 4) placed on these ends as X-ray markers, which consist of an X-ray-opaque material and are provided inside the balloon (7) on the inner tubing (2), wherein. between the inner tubing (2) and the outer balloon (7), tubing (5) forming an additional level and consisting of a soft-elastic material is provided as an intermediate layer in such a way that it extends in the longitudinal direction over the sleeves (3, 4) forming the X-ray markers and encloses an air cushion in the longitudinal direction between the sleeves (3, 4), **characterised in that** the air cushion is compressed when the stent is not expanded, and the tubing (5) has a larger outer diameter in this condition in the region of the sleeves (3, 4) than in its longitudinal portion between the sleeves (3, 4).

2. Balloon catheter as claimed in claim 1, **characterised in that** the inner diameter of the tubing (5) forming the intermediate layer is adapted to the outer diameter of the sleeves (3. 4) forming the X-ray markers in such a way that in the unloaded state it encloses an air cushion (6) towards the inner tubing (1).

3. Balloon catheter as claimed in claim 1 or 2, **characterised in that** the tubing (5) forming the intermediate layer is substantially of a thickness which corresponds to that of the sleeves (3, 4) forming the X-ray markers.

4. Balloon catheter as claimed in any one of the preceding claims, **characterised in that** the tubing (5) forming the intermediate layer reaches at least at one end beyond the sleeve (3) forming the X-ray marker.

5. Balloon catheter as claimed in any one of the preceding claims, **characterised in that** the tubing (5) forming the intermediate layer is attached only at its distal end to the catheter, in particular to the inner tubing (2).

6. Balloon catheter as claimed in any one of the preceding claims, [characterised in] that sleeves (3, 4) forming the X-ray markers form an air-tight closure so that a volume of air, which remains between the inner tubing (2) and the outer tubing (5), which forms the intermediate layer, forms an elastic air cushion (6) which, when the stent (9) is crimped during manufacture, forms a counter holding point for a controlled deflection of the balloon (7).

7. Balloon catheter as claimed in any one of the preceding claims, **characterised in that** the air cushion (6) is located in the longitudinal direction between the X-ray marking rings (3, 4) and is provided radially outside the inner tubing (2) and the X-ray marking rings (3, 4) but inside the balloon (7) and the stent (9) lying on the outside.

8. Balloon catheter as claimed in any one of the preceding claims, **characterised in that** the inner tubing (2), the balloon (7), the tubing (5), which forms the intermediate layer, and the stent (9) crimped onto this underlayer form a pre-assembled unit (1).

9. Balloon catheter as claimed in any one of the preceding claims, **characterised in that** the tubing (5), which forms the intermediate layer, consists of a soft and elastic material into which the stent (9) is pressed during crimping.

10. Balloon catheter as claimed in any one of the preceding claims, **characterised in that** the tubing (5), which forms the intermediate layer, has a Shore hardness of substantially 35D.

11. Balloon catheter as claimed in any one of the preceding claims, **characterised in that** the tubing (5), which forms the intermediate layer, is welded at the distal end to the inner tubing (2) of the catheter.

12. Balloon catheter as claimed in any one of the preceding claims, **characterised in that** the outer diameter of the catheter with the stent (9) crimped thereon has a value between the X-ray marking rings (3, 4) which is substantially smaller than the value of the catheter outer diameter at the sites where the marking rings (3, 4) are attached.

13. Balloon catheter as claimed in any one of the preceding claims, **characterised in that** the tubing (5), which forms the intermediate layer, is attached outside the balloon (7), the X-ray marking rings (3. 4) and the catheter inner tubing (2), and the stent (9) is compressed thereon.

## Revendications

1. Cathéter à ballonnet (1) pour élargir des rétrécissements vasculaires et pour la mise en place simultanée d'un stent déformable durablement (9) dans le vaisseau à élargir pour stabiliser celui-ci dans l'état élargi, dans lequel :
- la région distale du cathéter prévue pour recevoir le stent présente un tuyau flexible intérieur (2) qui est entouré par le stent non expansé ;
- un ballonnet (7) est agencé entre le stent (9) et le tuyau flexible intérieur (2) ;
- le tuyau flexible intérieur présente sur ses extrémités deux douilles (3, 4) placées sur celles-ci à titre de marques radiographiques qui sont prévues en un matériau radio-opaque à l'intérieur du ballonnet (7) sur le tuyau flexible intérieur (2), et
- un tuyau flexible (5) formant un plateau supplémentaire et se composant d'un matériau élastique mou est prévu à titre de couche intermédiaire entre le tuyau flexible intérieur (2) et le ballonnet extérieur (7) de manière à s'étendre en direction longitudinale jusqu'au-delà des douilles (3, 4) formant les marques radiographiques et de manière à enfermer en direction longitudinale une poche d'air entre les douilles (3, 4),
**caractérisé en ce que** la poche d'air est comprimée dans le stent non expansé et **en ce que**, dans la région des douilles (3, 4), le tuyau flexible (5) a dans cet état un diamètre extérieur plus grand que dans son tronçon longitudinal disposé entre les douilles (3, 4).

2. Cathéter à ballonnet selon la revendication 1, **caractérisé en ce que** le diamètre terminal intérieur du tuyau flexible (5) formant la couche intermédiaire est adapté au diamètre extérieur des douilles (3, 4) formant les marques radiographiques de manière à enfermer dans l'état non chargé une poche d'air (6) vers le tuyau flexible intérieur (2).

3. Cathéter à ballonnet selon l'une ou l'autre des revendications 1 et 2, **caractérisé en ce que** le tuyau flexible (5) formant la couche intermédiaire présente essentiellement une épaisseur qui correspond à celles des douilles (3, 4) formant les marques radiographiques.

4. Cathéter à ballonnet selon l'une des revendications précédentes, **caractérisé en ce que** le tuyau flexible (5) formant la couche intermédiaire s'étend au moins sur une extrémité au-delà de la douille (3) formant une marque radiographique.

5. Cathéter à ballonnet selon l'une des revendications précédentes, **caractérisé en ce que** le tuyau flexible (5) formant la couche intermédiaire est fixé au cathéter, en particulier au tuyau flexible intérieur (2), seulement sur son extrémité distale.

6. Cathéter à ballonnet selon l'une des revendications précédentes, **caractérisé en ce que** les douilles (3, 4) formant les marques radiographiques forment une terminaison étanche à l'air, de sorte qu'un volume d'air qui reste entre le tuyau flexible intérieur (2) et le tuyau flexible extérieur (5) formant la couche intermédiaire, forme un coussinet d'air élastique (6) qui, lors du sertissage du stent dans le cadre de la fabrication, forme un support pour une déviation contrôlée du ballonnet (7).

7. Cathéter à ballonnet selon l'une des revendications précédentes, **caractérisé en ce que** le coussinet d'air (6) se trouve en direction longitudinale entre les bagues de marquage radiographique (3, 4), et **en ce que** ledit coussinet d'air est prévu radialement en dehors du tuyau flexible intérieur (2) et des bagues de marquage radiographique (3, 4), mais à l'intérieur du ballonnet (7) et du stent (9) disposé à l'extérieur.

8. Cathéter à ballonnet selon l'une des revendications précédentes, **caractérisé en ce que** le tuyau flexible intérieur (2), le ballonnet (7), le tuyau flexible (5) formant la couche intermédiaire, et le stent (9) serti sur cette couche sous-jacente forment une unité prémontée (1).

9. Cathéter à ballonnet selon l'une des revendications précédentes, **caractérisé en ce que** le tuyau flexible (5) formant la couche intermédiaire se compose d'un matériau mou et élastique dans lequel le stent (9) est enfoncé lors du sertissage.

10. Cathéter à ballonnet selon l'une des revendications précédentes, **caractérisé en ce que** le tuyau flexible (5) formant la couche intermédiaire présente une dureté Shore essentiellement de 35 D.

11. Cathéter à ballonnet selon l'une des revendications précédentes, **caractérisé en ce que** le tuyau flexible (5) formant la couche intermédiaire est soudé sur l'extrémité distale au tuyau flexible intérieur (2) du cathéter.

12. Cathéter à ballonnet selon l'une des revendications précédentes, **caractérisé en ce que** le diamètre extérieur du cathéter avec stent serti (9) présente, entre les bagues de marquage radiographique (3, 4), une valeur qui est sensiblement égale ou inférieure à la valeur du diamètre extérieur du cathéter aux emplacements où sont apposées les bagues de marquage (3, 4).

13. Cathéter à ballonnet selon l'une des revendications précédentes, **caractérisé en ce que** le tuyau flexible (5) formant la couche intermédiaire est monté en dehors du ballonnet (7), des bagues de marquage radiographique (3, 4) et du tuyau flexible intérieur (2) du cathéter et **en ce que** le stent (9) est compressé sur celui-ci.
